# EUROPEAN PATENT SPECIFICATION

(11) **EP 0 665 039 B1**
(45) Date of publication and mention of the grant of the patent: **01.09.1999**
(21) Application number: 95300270.6
(22) Date of filing: 17.01.1995
(51) Int. Cl.: A63F 9/22, G09B 9/00

(54) **Enhancement of virtual reality effect using fragrance**
Verbesserung des virtuellen Realitätseffekts durch Einsatz von Duftstoffen
Amélioration d'effet de réalité virtuelle en utilisant du parfum

(30) Priority: 01.02.1994 GB 9401899
(43) Date of publication of application: 02.08.1995
(73) Proprietor: The BOC Group plc, Windlesham Surrey GU20 6HJ (GB)
(72) Inventor: Shervington, Evelyn Arthur, Nr Petersfield, Hampshire, GU31 5LD (GB)
(74) Representative: Gough, Peter

(56) References cited:
- EP-A- 0 258 619
- EP-A- 0 508 939
- WO-A-92/09949
- FR-A- 2 597 190
- US-A- 4 952 024
- IEE COLLOQUIUM ON 'REAL WORLD VISUALISATION - VITUAL WORLD - VIRTUAL REALITY' (DIGEST NO.197), 26 September 1991 LONDON, UK , pages 9/1-9/3, G. ALLPORT 'Three dimensional navigation using the five senses'

## Description

The present invention relates to simulation apparatus and in particular to apparatus found in "virtual reality" technology. Virtual reality systems usually consist of a computer control module, a headset worn by a user and a joy stick and/or sensitive gloves. Three-dimensional computer graphics are projected inside the headset and produce a pre selected view of, for example, a race track. Computerised vehicles move along the track and their speed and direction can be controlled by movements of the joy stick/gloves. The view can be changed with every movement of the users headset or joy stick/gloves. It is also known to fit the headset with a stereo system so that movements of the headset can alter the volume and direction of sound to simulate the noise of a vehicle as it moves along a race track.

In an article entitled "Three-dimensional navigation using five senses by G.Allport published on 26^{th} September, 1991 there is disclosed a virtual reality system which includes the production of smells to simulate the real world. However, there is no detail as to how the smell or smells are produced and delivered to the user or how the smells are controlled.

US Patent No 4952024 describes a virtual reality apparatus which include a headset to which smells can be delivered which smells emanate from scent pallets.

French Patent application 2597190 discloses a simulation apparatus in which the aromagas comprises an ingredient or ingredients for creating the smell or fragrance dissolved in a solvent and stored under pressure in a gas cylinder. A solenoid valve is provided for computer controlled release of the aromagas.

It is an aim of the present invention to enhance the reality of the "virtual reality" effect by delivering one or more smells or fragrances in a controlled manner to the headset or other computer graphics signal receiving means.

According to the present invention, a simulation apparatus includes a computer control module (2), receiving means for receiving three-dimensional computer graphics from said computer control module and means for providing a smell or fragrance in the form of an aromagas at the receiving means, which is characterised by comprising a solenoid valve (12) and in that the aromagas is initially contained in a cylinder under pressure, the flow of aroma gas from the cylinder towards the receiving means being controlled by the solenoid valve which is operated at pre-selected intervals and for pre-selected durations by a signal or signals from the computer control module.

Preferably, the means for receiving three-dimensional computer graphics is a headset. Preferably, the aromagas consists of a particular ingredient for creating the smell or fragrance dissolved in a solvent and stored under pressure in a gas cylinder, a gas line is provided in fluid communication between the gas cylinder and the headset and a solenoid valve controls the release of the aromagas from the cylinder to the headset.

In a preferred embodiment the solenoid valve is operated at preselected intervals and preselected durations by signals emanating from the computer control module.

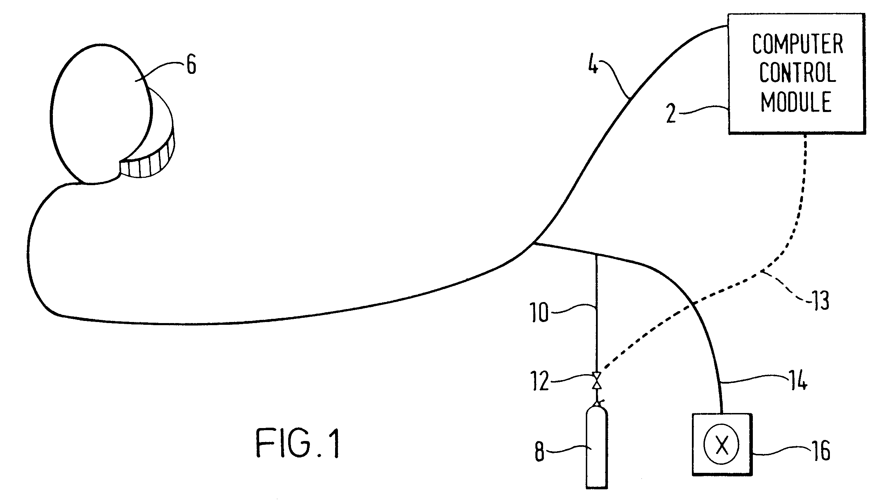

An embodiment of the invention will now be described, by way of example, reference being made to the Figure of the accompanying diagrammatic drawing which is a schematic view of a simulator apparatus.

As shown a simulator apparatus for use as a "virtual reality" computerised entertainment system comprises a computer control module 2 connected by means of a control cable 4 in a manner known per se to a headset 6. Arranged in parallel with the control cable 4 for at least a portion of its length is an aromagas line 10 in the form of a tube having a small bore, for example, 20 gauge bore said tube extending from a gas cylinder 8 to the headset 6. The gas cylinder 8 contains under high pressure, in the order of 50 bar, an active ingredient, that is, a substance which on release from the cylinder 8 will create the desired smell/fragrance dissolved in a solvent, for example, carbon dioxide and alcohol and is herein referred to as the "aromagas".

The flow of the aromagas from the cylinder 8 is controlled by a liquid/gas solenoid valve 12. As shown, there is provided a control cable 13 extending from the solenoid valve to the computer control module 2.

Also in flow communication with the aromagas line 10 is an air line 14 extending from an air compressor 16.

In use, during a "virtual reality" game, according to the programme being operated, at selected intervals and for selected durations the computer control module 2 will send a signal or signals to the solenoid valve 12 to allow a bolus of aromagas to leave the cylinder 8, enter the aromagas line 10 and flow towards the headset 6. At a preselected interval after each bolus has reached the headset 6 air under pressure from the air compressor 16 flows through the air line 14, and aromagas line 10 to purge the aromagas from the line 10 and eventually the headset 6.

For safety reasons, there are limits to the duration of smell "boluses", that is to say the size of bolus will be limited so that the player could not be asphyxiated and there will also be controls as to the frequency of such boluses. This may be done through a timer override device to ensure that the boluses of aromagas arrive at the headset, for example, at an interval of one per minute.

Although reference has been made in the above described embodiment to one gas cylinder containing aromagas, it is envisaged that a range of smells and fragrances could be provided to the headset 6 by means of a plurality of gas cylinders 8.

A small computer control unit would be provided to operate the solenoid valve of each gas cylinder 8 at a predetermined time to ensure that the appropriate smell arrived at the headset at the appropriate time.

Each gas cylinder 8 would have its own aromagas line each taking a separate fragrance to the headset. In this embodiment the air purge would then entrain via a venturi device each bolus of aromagas as required.

Alternatively, a single hose line would connect with the headset attached to the electrical control cable 4 and the various boluses of aromagas gas would follow each other up the hose as demanded and these could be placed under pressure by means of the air line 14.

Although reference has been made to virtual reality games it is envisaged that this system of supplying a headset with aromagas could be applied to more serious training situations where, for example, flight simulation is used to train pilots.

## Claims

1. A simulation apparatus including a computer control module (2), receiving means (6) for receiving three-dimensional computer graphics from said computer control module (2) and means for providing a smell or fragrance in the form of an aromagas at the receiving means (6), characterised by comprising a solenoid valve (12) and in that the aromagas is initially contained in a cylinder under pressure, the flow of aromagas from the cylinder towards the receiving means (6) being controlled by the solenoid valve (12) which is operated at pre-selected intervals and for pre-selected durations by a signal or signals from the computer control module (2).

2. A simulation apparatus as claimed in claim 1, in which the aromagas comprises an ingredient or ingredients for creating the smell or fragrance dissolved in a solvent and stored under pressure in the cylinder (8), and in that an aromagas line (10) is produced for fluid communication between the cylinder (8) and the receiving means (6) the solenoid valve (12) being located in the aroma gas line (10).

3. A simulation apparatus as claimed in claim 2, in which an air line (14) is provided said air line (14) being in fluid communication between an air compressor (16) and the aromagas line (10) for purging aromagas from the aromagas line and the receiving means (6) at preselected intervals of time.

4. A simulation apparatus as claimed in claim 1,2 or 3, in which a plurality of gas cylinders are provided each containing a different aromagas each being connected by an individual aromagas line to the headset.

5. A simulation apparatus as claimed in claim 4, in which a solenoid valve is associated with each aromagas cylinder said solenoids being operated by a computer control unit such that each aromagas arrives at the receiving means (6) at a preselected time and remains there for a preselected interval of time.

6. A simulation apparatus as claimed in claims 4 or 5, in which each aromagas cylinder is connected to the receiving means 6 by means of a single hose line.

## Patentansprüche

1. Simulationsgerät mit einem Computersteuermodul (2), Empfangsmitteln (6) für den Empfang dreidimensionaler Computergraphiken von dem genannten Computersteuermodul (2), und Mitteln zum Erzeugen eines Geruchs oder Dufts in Form eines Aromagases an den Empfangsmitteln (6), dadurch gekennzeichnet, daß es ein Elektromagnetventil (12) aufweist und daß das Aromagas anfänglich in einem Zylinder unter Druck enthalten ist und die Aromagasströmung vom Zylinder zu den Empfangsmitteln (6) durch das Elektromagnetventil (12) gesteuert wird, das in vorgewählten Intervallen und während vorgewählter Zeiten durch ein Signal oder durch Signale vom Computersteuermodul (2) betätigt wird.

2. Simulationsgerät nach Anspruch 1, wobei das Aromagas einen Bestandteil oder Bestandteile zur Erzeugung des Geruchs oder Duft in einem Losungsmittel aufgelöst und unter Druck in den Zylinder (8) gespeichert enthält, und daß eine Aromagasleitung (10) zur Strömungsverbindung zwischen dem Zylinder (8) und den Empfangsmitteln (6) hergestellt ist und das Elektromagnetventil (12) in der Aromagasleitung (19) angeordnet ist.

3. Simulationsgerät nach Anspruch 2, wobei eine Luftleitung (14) vorgesehen ist, die in Strömungsverbindung zwischen einem Luftkompressor (16) und der Aromagasleitung (10) verläuft, um Aromagas aus der Aromagasleitung und aus den Empfangsmitteln (6) in vorgewählten Zeitintervallen auszuspülen.

4. Simulationsgerät nach Anspruch 1, 2 oder 3, wobei eine Mehrzahl von Gaszylindern vorgesehen ist, die jeweils ein anderes Aromagas enthalten und die jeweils durch eine eigene Aromagasleitung mit der Kopfeinheit verbunden sind.

5. Simulationsgerät nach Anspruch 4, wobei ein Elektromagnetventil jedem Aromagaszylinder zugeordnet ist und die Elektromagnete durch eine Computersteuereinheit betätigt werden, derart, daß jedes Aromagas bei den Empfangsmitteln (6) zu einer vorgewählten Zeit ankommt und dort während eines vorgewählten Zeitintervalls verbleibt.

6. Simulationsgerät nach Anspruch 4 oder 5, wobei jeder Aromagaszylinder mittels einer Einfachschlauchleitung mit den Empfangsmitteln (6) verbunden ist.

## Revendications

1. Dispositif de simulation comprenant un module (2) de commande par ordinateur, des moyens récepteurs (6) pour la réception d'une infographie tridimensionnelle en provenance dudit module (2) et des moyens pour fournir une odeur ou une fragrance sous forme d'un gaz aromatisé aux moyens récepteurs (6), ***caractérisé en ce qu***'il comprend une vanne à solénoïde (12) et ***en ce que*** le gaz aromatisé est initialement contenu dans une bouteille sous pression, l'écoulement du gaz aromatisé de la bouteille jusqu'aux moyens récepteurs (6) étant régulé par la vanne à solénoïde (12) qui est actionnée à intervalles présélectionnés et pour des durées présélectionnées par un signal ou des signaux en provenance du module (2) de commande par ordinateur.

2. Dispositif de simulation selon la Revendication 1, dans lequel le gaz aromatisé comprend un ingrédient ou des ingrédients destiné(s) à créer l'odeur ou la fragrance, dissous dans un solvant et stocké(s) sous pression dans la bouteille (8), et dans lequel une canalisation (10) pour le gaz aromatisé est réalisée pour assurer une communication fluidique entre la bouteille (8) et les moyens récepteurs (6), la vanne à solénoïde (12) étant située dans la canalisation (10) pour le gaz aromatisé.

3. Dispositif de simulation selon la Revendication 1, dans lequel une canalisation (14) à air est présente, ladite canalisation (14) à air étant en communication fluidique entre un compresseur (16) d'air et la canalisation (10) pour le gaz aromatisé, dans le but de purger le gaz aromatisé de la canalisation pour le gaz aromatisé et les moyens récepteurs (6) à intervalles de temps présélectionnés.

4. Dispositif de simulation selon la Revendication 1, 2 ou 3, dans lequel une pluralité de bouteilles de gaz sont présentes, contenant chacune un gaz aromatisé différent, chacune raccordée au casque par une canalisation pour gaz aromatisé individuelle.

5. Dispositif de simulation selon la Revendication 4, dans lequel une vanne à solénoïde est associée à chaque bouteille de gaz, lesdits solénoïdes étant actionnés par une unité de commande informatique de telle sorte que chaque gaz aromatisé arrive aux moyens récepteurs (6) à un moment présélectionné et y demeure pendant un intervalle de temps présélectionné.

6. Dispositif de simulation selon la Revendication 4 ou 5, dans lequel chaque bouteille de gaz aromatisé est reliée aux moyens récepteurs (6) par l'intermédiaire d'une canalisation unique en tuyau souple.
